**Europäisches Patentamt**

**European Patent Office**

**Office européen des brevets**

(19)

(11) Publication number: **0018199**
**A1**

(12)

# EUROPEAN PATENT APPLICATION

(21) Application number: **80301212.9**

(22) Date of filing: **17.04.80**

(51) Int. Cl.³: **A 61 K 7/06**

(30) · Priority: **17.04.79 ZA 791787**

(71) Applicant: **Pegg, Keith John Stephenson, 6 Saltburn Close, Mount Pleasant Salisbury (RH)**
Applicant: **Pegg, Luciele Violet, 6 Saltburn Close, Mount Pleasant Salisbury (RH)**

(43) Date of publication of application: **29.10.80**
**Bulletin 80/22**

(72) Inventor: **Pegg, Keith John Stephenson, 6 Saltburn Close, Mount Pleasant Salisbury (RH)**
Inventor: **Pegg, Luciele Violet, 6 Saltburn Close, Mount Pleasant Salisbury (RH)**

(84) Designated Contracting States: **AT BE CH DE FR GB IT LI LU NL SE**

(74) Representative: **Roberts, Peter William, Marks & Clerk Alpha Tower ATV Centre, Birmingham B1 1TT (GB)**

(54) **Hair conditioning preparations.**

(57) A conditioning preparation for application to the human scalp to promote hair growth or at least minimise the loss of hair includes a base material and 5 to 15% of vitamin E oil and 5 to 15% of almond oil. Commonly known herbal ingredients and perfume additives may also be included in the conditioning preparation. One particular preparation includes 60 to 88% of a base material comprising an homogenized mixture of 80% cholesterol cream and 20% bees wax, 5 to 15% of vitamin E oil, 5 to 15% of almond oil, and 2 to 10% of powdered camomile flowers.

0018199

The invention relates to conditioning preparations and more particularly to conditioning preparations to be applied to the human scalp.

A great many conditioning preparations are on the market and for many of these claims are mode ·to promote hair growth or at least to minimise loss of hair.

This invention is concerned with preparations of this nature.

According to one aspect of the invention, a conditioning preparation includes a base material together with 5 to 15 per cent of vitamin 'E' oil and 5 to 15 per cent of almond oil.

According to a further aspect, a conditioning preparation suitable for application to the human scalp includes a quantity of base material to which is added from 5 to 15 per cent of vitamin 'E' oil and from 5 to 15 per cent of almond oil.

Preferably, the preparation contains 7 per cent of each of the vitamin 'E' oil and almond oil.

According to yet a further aspect of the invention, a conditioning preparation suitable for application to the human scalp includes from 60 per cent to about 88 per cent of a base material comprising an homogenised mixture of cholesterol cream with protein and bees wax in the ratio of about 80 per cent cholesterol cream with protein to about 20 per cent bees wax; to which base is added from 5 to 15 per cent vitamin 'E' oil, from 5 to 15 per cent of almond oil and from 2 to 10 per cent of powdered camomile flowers.

0018199

Preferably, the vitamin 'E' oil and almond oil each comprise 10 per cent while the powdered camomile flowers comprise 5 per cent weight in volume of the total mixture.

In order to illustrate the invention a series of suitable base formulations will now be described as follows:-

BASE 1.

| | | |
|---|---|---|
| Cetostearyl alcohol | 5.8 | per cent |
| Triethanolamine | 1.5 | per cent |
| Nipigin M | 0.1 | per cent |
| Cetrimide | 0.5 | per cent |
| add Water | 92.1 | per cent |

BASE 2.

| | | |
|---|---|---|
| Stearic acid | 20.9 | gm |
| Cetyl alcohol | 2.9 | gm |
| Triethanolamine | 2.0 | ml |
| Propylene glycol | 10.0 | ml |
| Light liquid paraffin | 2.0 | ml |
| Borax | 14.9 | gm |
| Water | 450.0 | ml |
| Nipigin M | 0.7 | gm |

Heat together the stearic acid, cetyl alcohol, liquid paraffin, triethanolamine, propylene glycol with the water and then add the nipigin M and borax dissolved in hot water. Finally 10 ml of a strong ammonia solution is added /and, the mixture is stirred until homogeneous. The mixture is then cooled and further water is added, with stirring, to achieve the required volume.

BASE 3

| | |
|---|---|
| Stearyl alcohol | 18 parts by weight |
| Cetyl alcohol | 4 parts by weight |
| Triethanolamine | 2 parts by weight |
| Glycerol | 5 parts by weight |
| Water | 71 parts by weight |

The stearyl alcohol and cetyl alcohol are melted together while the triethanolamine and glycerol are dissolved in the water and heated to 80°C. To the solution is added the melted stearyl and cetyl alcohols with constant stirring until cool.

BASE 4

| | |
|---|---|
| Stearic acid | 160 gm |
| Water | 720 ml |

Heat together until boiling and then add the following ingredients which have been premixed.

| | |
|---|---|
| Strong ammonia solution | 10 ml. |
| Water | 160 ml |
| Glycerine | 20 ml |

Stir until cool and add 0.1 gm nipigin M as a preservative.

BASE 5

| | |
|---|---|
| Cholesterol cream | 80 per cent mixed with 20 per cent bees wax |

heated and homogenised.

0018199

BASE 6

| | |
|---|---|
| Cholesterol cream or | |
| Lanolin | 50 per cent |
| Vitamin 'E' oil | 25 per cent |
| Almond oil or | |
| Olive oil | 25 per cent |

BASE 7

| | |
|---|---|
| Cetyl alcohol | 15 gm |
| Glycerol | 5 gm |
| Sodium Lauryl Sulphate | 1.5 gm |
| Water | 200.0 ml |

Melt the alcohol and glycerol together at 70°C.
Dissolve the sodium lauryl sulphate in the water and heat
to 70° C.      Mix slowly and stir until cool.

BASE 8

| | |
|---|---|
| Cetyl alcohol | 15 gm |
| Glycerol | 5 gm |
| Cetyl Methylammonium | |
| Chloride | 1.5 gm |
| Water | 200 ml |

Melt alcohol and glycerol together at 70° C
dissolve the cetyl methylammonium chloride in the water
and heat to 70°C.   Mix the two together slowly and stir
until cool.

Each of the above bases, except for base 6, is formulated into a conditioning preparation suitable for application to the human scalp by adding to, and amalgamating with, the base 5 to 15%, or more preferably 7%, of vitamin E and 5 to 15%, or more preferably 7% of almond oil.   In the case of base 6, the concentration of vitamin E oil or the concentration of vitamin E oil and almond oil must be decreased when the base is formulated into a conditioning preparation, dilution conveniently being effected by adding additional colesterol cream or lanolin.

In addition to vitamin E oil and almond oil, any of the herbal ingredients known to assist in or promote hair growth or reduce the fall of hair may be included in the conditioning preparation.   Examples of suitable herbal ingredients include camomile flowers or powdered camomile flowers, tincture of marigold, birth  T, nettle extract (urtica dioica and urtica urens).   Thus, a preferred conditioning preparation includes from 60 to 88% of a base material comprising a homogenized mixture of cholesterol cream and bees wax in the ratio of about 80% cholesterol cream to about 20% bees wax, 5 to 15%, or more preferably 10%, of vitamin E oil, 5 to 15%, or more preferably 10%, of almond oil and 2 to 10%, or more preferably 5%, of powdered camomile flowers.   Such a mixture would require thorough dispersion by shaking prior to application to the scalp.

Further additives providing a perfume content, such as lavender oil, rosemary oil and sesame oil may also be incorporated in the conditioning preparation.

0018199

C L A I M S:-

1. A conditioning preparation including a base material together with 5 to 15 per cent of vitamin 'E' oil and 5 to 15 per cent of almond oil.

2. A conditioning preparation suitable for application to the human scalp including a quantity of base material to which is added from 5 to 15 per cent of vitamin 'E' oil and from 5 to 15 per cent of almond oil.

3. A conditioning preparation as claimed in claim 1 or claim 2 wherein the vitamin 'E' oil and almond oil each constitute 7 per cent of the total mixture.

4. A conditioning preparation suitable for application to the human scalp including about 60 to 88 per cent of a base material comprising an homogenised mixture of cholesterol cream with protein and bees wax in the ratio of about 80 per cent cholesterol cream with protein to about 20 per cent bees wax; to which base is added 5 to 15 per cent of vitamin 'E' oil, from 5 to 15 per cent of almond oil and from 2 to 10 per cent of camomile powdered flowers.

5. A conditioning preparation as claimed in claim 4 wherein the base material comprises 75 per cent of the total mixture, the vitamin 'E' and almond oils, each comprise 10 per cent, while the powdered camomile flowers comprise 5 per cent weight in volume of the total mixture.

0018199

6.    A conditioning preparation as claimed in any preceding claim in which the base material is selected from any of the Bases 1 to 8 as hereinbefore described.

| Category | Citation of document with indication, where appropriate, of relevant passages | Relevant to claim | CLASSIFICATION OF THE APPLICATION (Int. Cl. 3) |
|---|---|---|---|
| A | GB - A - 1 466 062 (K.J. KNIGHT)<br>* Page 1, lines 16-36; example 1; claim 1 * | 1 | A 61 K 7/06 |
| A | CH - A - 272 709 (P. EMPIA-CAHERA)<br>* Examples * | 1,4 | |
| A | DE - A - 2 145 204 (E.S. MAUGHAM<br>* Example 1; claims 1,10,14,15 * | 1 | TECHNICAL FIELDS SEARCHED (Int.Cl. 3) |
| A | FR - A - 1 249 468 (Mme SEBILLEAU)<br>* Abstract * | 1 | A 61 K 7/06 |
| A | FR - A - 2 303 526 (P. FABRE S.A.)<br>* Claims * | 4 | |

**DOCUMENTS CONSIDERED TO BE RELEVANT**

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 25-07-1980 | WILLEKENS |

EPO Form 1503.1 06.78